# EUROPEAN PATENT APPLICATION

(11) **EP 1 077 258 A1**
(43) Date of publication of application: **21.02.2001**
(21) Application number: 99918278.5
(22) Date of filing: 28.04.1999
(51) Int. Cl.: C12N 15/12, C07K 14/82, C07K 16/18

(54) **HUMAN HRPI**

(30) Priority: 30.04.1998 JP 12024698
(71) Applicant: Sumitomo Electric Industries, Ltd., Osaka-shi, Osaka 541-0041 (JP)
(72) Inventor: KISHIMOTO, Toshihiko, Yokohama-shi, Kanagawa 244-8588 (JP); NIWA, Shin-ichiro, Yokohama-shi, Kanagawa 244-8588 (JP); NISHIKAWA, Kazuko, Yokohama-shi, Kanagawa 244-8588 (JP)
(74) Representative: Baldock, Sharon Claire
(86) International application number: JP9902314
(87) International publication number: WO9957269

(57) **Abstract**

The base sequence of human HRPI gene, which exhibits remarkable expression in hepatic cancer and regulates cell cycle and cell division, is clarified. Furthermore, disclosed are the polynucleotides (including human HRPI gene) comprising the base sequence, human HRPI proteins encoded by the polynucleotides, and antibodies recognizing the HRPI proteins.

## Description

### Technical Field

The present invention relates to proteins which are expressed markedly in hepatic cancer and which control cell cycle and cell division, genes encoding the proteins, and the antibodies to the proteins.

### Background Art

It is known that carcinoma or cancer occurs because of an abnormality in a gene. A change abnormality in a gene at its transcription level, in particular, is regarded as a major cause of cancer. In order to elucidate the mechanism of the onset of carcinoma, the acquisition of proteins variedly expressed during carcinogenesis and genes encoding the proteins, or the acquisition of proteins different in the state of expression among tissues and genes encoding the proteins has been performed actively since about 1980.

In PCT International Application Publication No. WO 97/10333 the inventors disclosed the sequence of HRPI protein as a novel protein which increases in expression during a carcinogenic process as well as HRPI gene as a novel gene which encodes the protein, by isolating and identifying them by utilizing a subtraction method.

### Disclosure of the Invention

The purpose of the present invention is to identify an amino acid sequence of human HRPI protein and the base sequence of the gene which encodes the protein, and thereby to prepare and detect the protein and the gene by means of a genetic engineering technique, and furthermore to provide an antibody to the protein.

The present invention discloses the base sequence of the human HRPI gene and provides a polynucleotide consisting of the base sequence.

The present invention also provides an antisense polynucleotide consisting of the complementary base sequence corresponding to the base sequence.

A method of preparing human HRPI protein (hereinafter sometimes referred to as "human HRPI") is also disclosed in the present patent application. More specifically, disclosed is a method by which human HRPI is prepared by a transformant having human HRPI cDNA introduced therein. Furthermore, human HRPI prepared by such preparation method is disclosed. Further disclosed are a preparation method of the mutants of human HRPI prepared by substitution, deletion or addition of one or more amino acids in the amino acid sequences of human HRPI and the mutants of human HRPI prepared by using such preparation method.

Moreover, the present invention discloses an antisense polynucleotide consisting of the complementary base sequence corresponding to the base sequence of the polynucleotide encoding the above-mentioned human HRPI or the polynucleotide encoding a human HRPI mutant. Although an antisense polynucleotide is included in the category of polynucleotide, the term "antisense polynucleotide" is used, in this specification, to specify that a polynucleotide consists of the base sequence of an antisense strand. Antisense DNA and antisense RNA are representative of the antisense polynucleotide.

The present invention further discloses a whole or part of the polynucleotide which encodes human HRPI or a human HRPI mutant and the part of the polynucleotide comprises at least 12 bases.

As for the polynucleotide, its coding regions can be used for preparing a partial length protein of human HRPI or a human HRPI mutant, respectively. It can also be used as a probe.

The present invention also discloses a whole or part of the antisense polynucleotide corresponding to a polynucleotide which encodes human HRPI or a human HRPI mutant and the antisense polynucleotide comprises at least 12 bases.

This antisense polynucleotide can inhibit the biosynthesis of human HRPI or the human HRPI mutant respectively. It can also be used as a probe.

The present invention discloses a derivative of the polynucleotide (including an antisense polynucleotide).

The present invention also discloses a polynucleotide (including an antisense polynucleotide) that is chemically modified.

Furthermore, the present invention provides an antibody that recognizes human HRPI and a mutant of the human HRPI.

In the present patent application, the antigenicity of human HRPI, i.e. the fact that the antibody can be made from human HRPI is disclosed.

### Brief Description of the Drawing

Figure 1 shows the result of electrophoresing human HRPI.
Figure 2 shows the result of Western blotting human HRPI with the antiserum that was obtained by administrating human HRPI to a rabbit.
Figure 3 shows the result of examining the expression of human HRPI mRNA in each human tissue by Northern blotting.

### Best Mode for Carrying out the Invention

With the human HRPI gene of the present invention a transformant can be prepared by inserting it into a suitable vector (e.g., TA cloning vector) and then introducing it to a host (e.g., E. coli). Then, by culturing the transformant, human HRPI can be prepared.

By a naturally occurring mutation or an artificial mutation (e.g., the method described in Molecular Cloning 2nd Edition, pages15.1-15.113), it is possible to change a polynucleotide without altering the main function of the polypeptide that the polynucleotide encodes. By this method, it is possible to prepare mutants of human HRPI for the human HRPI of the present invention. That is, it is possible to prepare proteins that consist of the amino acid sequences in which one or more amino acids set forth in SEQ ID NO: 1 of the Sequence Listing are substituted or deleted, or in which one or more amino acids are added to the amino acid sequences of SEQ ID NO: 1 of Sequence Listing. Here, the homology of amino acids between the human HRPI proteins and the mutants thereof is preferably from 75 % to less than 100 %.

The amino acid sequence of a mutant of the human HRPI protein according to the present invention can be determined from the base sequence of the gene that encodes the mutant. This is possible by means of a program available in the market, e.g., Genetix-Mac (Registered trademark, Software Development Inc.).

It is possible, by the degeneracy of a genetic codon, to substitute at least partly bases of the base sequence of the polynucleotide with other kinds of bases without changing the amino acid sequence of the polypeptide translated from the polynucleotide. Therefore, the polynucleotide of the present invention that encodes human HRPI includes all patterns of the degeneracy.

DNA that consists of the base sequence set forth in SEQ ID NO: 2 of the Sequence Listing is human HRPI cDNA that exists in nature.

The present invention includes the antisense polynucleotide consisting of the base sequence of the antisense strand of the polynucleotide (including a noncoding region) that encodes the above-mentioned human HRPI, and the derivative thereof. The antisense polynucleotide can be hybridized with the polynucleotide that encodes human HRPI, and can inhibit the biosynthesis of the polypeptide that the polynucleotide encodes, if the polynucleotide to which it hybridizes is the polynucleotide of the coding region.

The antisense polynucleotide for inhibiting the biosynthesis of the polypeptide preferably consists of 15 or more bases. On the other hand, it is unsuitable to incorporate the full length of an antisense polynucleotide into the cell because it is too long. In the case of incorporating an antisense polynucleotide in the cell and inhibiting the biosynthesis of the human HRPI protein, the antisense polynucleotide used therefor should consist of 12 or more bases but not exceeding 30 bases, preferably 15 or more bases but not exceeding 25 bases, and more preferably 18 or more bases but not exceeding 22 bases.

The antisense polynucleotide or the part thereof according to the present invention, which is the combination of plural nucleotides consisting of a base, phosphate, and sugar, include all kinds of antisense polynucleotides, including those that do not exist in nature. They are typically antisense DNA and antisense RNA.

With respect to the antisense polynucleotides of the present invention, various antisense polynucleotide derivatives of high quality in terms of binding capacity with target DNA and RNA, tissue selectivity, cell permeation, nuclease resistance, and stability in the cell can be obtained, using the antisense techniques known in the art.

For ease of hybridizing, it is generally recommended to design the antisense polynucleotide or the derivative thereof that has a base sequence complementary to the base sequence of the region forming the loop of RNA.

The antisense polynucleotides that have sequences complementary to the sequences of the translation start codon neighborhood, the ribosomal binding site, the capping site, and the splicing site can generally be expected to give a high inhibition effect against expression. Therefore, the antisense polynucleotides or the derivatives thereof according to the present invention that include complementary sequences corresponding to the sequences of the translation start codon vicinity, the ribosomal binding site, the capping site, and the splicing site of the gene or RNA encoding human HRPI are expected to give a high inhibition effect against expression. As for the derivatives generally known in the art at present, the derivatives that are enhanced with respect to at least any one of the nuclease resistance, the tissue selectivity, the cell permeation, and the binding capacity are preferable. Especially preferable is a derivative that has a phosphorothioate bond as the skeleton structure. The derivatives that have these features or structure are included as the polynucleotides and derivatives thereof according to the present invention.

The antisense polynucleotides of the wild type can be prepared by synthesizing, using a chemical synthesizer or by the PCR method that uses the gene encoding human HRPI as a template. There are derivatives that can be chemically synthesized, such as those of methylphosphonate type and the phosphorothioate type. In such case the desired antisense polynucleotide or the derivative can be obtained by manipulation according to the manual attached to the chemical synthesizer and purifying the resultant synthetic products by the HPLC method utilizing reverse phase chromatography or the like. The antisense polynucleotides or the derivatives thereof according to the present invention can also be made by the above-mentioned method.

The polynucleotide of the present invention, which encodes human HRPI, and the antisense polynucleotide thereof or a part thereof (a polynucleotide which consists of the base sequences of 12 or more continuous bases) are usable as a probe to screen the human HRPI gene from a cDNA library and the like. In this case those of 30 to 70 % GC content are suitable for use.

Here, a polynucleotide that consists of the base sequence of 15 or more bases is especially preferable. The polynucleotide used as the probe may be a derivative. Generally, a sequence having a number of bases more than the above-mentioned number of bases is recognized as a specific sequence. The cDNA libraries that are made from mRNA can preferably be used in a screening in which the probe is employed. A group of cDNA chosen from these cDNA libraries by random sampling can be used as a sample for screening. Also, those available in the market are usable.

For example, DNA that consists of the base sequence of 12 or more continuous bases in the base sequence set forth in SEQ ID NO: 2 of the Sequence Listing or the polynucleotide (antisense polynucleotide) that hybridizes with the DNA is usable as a probe to screen a human HRPI gene from the cDNA library and the like.

Furthermore, the polynucleotide of the present invention that encodes human HRPI, the antisense polynucleotide thereof or a part of the polynucleotides can be used as a probe to detect the tissues in which mRNA derived from the human HRPI gene is expressed, by carrying out Northern blot hybridization with respect to mRNA derived from the respective tissues.

It is generally known to modify chemically, such as methylating a side chain, biotinylating or substituting by sulfer with oxygen at the phosphate portion, when chemically synthesizing DNA or RNA.

For example, when synthesizing DNA set forth in SEQ ID NO: 2 of the Sequence Listing, it is possible to chemically modify and to synthesize one that is different from the DNA itself shown in the Sequence Listing.

It is also possible to label cDNA with the radioactive isotope even if it is acquired from the cDNA library.

Therefore, DNA and RNA of the present invention include, as the scope, the above-mentioned chemically modified DNA, RNA or antisense polynucleotide. The chemically modified DNA or RNA is capable of displaying the function to encode a protein or the function as a probe and the chemically modified antisense polynucleotide is capable of displaying both the functions to inhibit the biosynthesis of the proteins and as a probe.

It is possible to introduce a plasmid into a suitable host such as E. coli and thereby to obtain a transformant with a known method. It is possible to prepare human HRPI protein or the mutant of the human HRPI protein by culturing the transformant into which the human HRPI gene of the present invention is introduced and allowing it to amplify the gene or to express the protein. It is possible to purify the human HRPI protein or the mutant of the human HRPI protein according to the present invention by recovering the resultant product and performing manipulations, according to need, such as concentration, solubilization, dialysis, and various chromatographies.

It is possible to prepare the human HRPI protein or the mutant of the human HRPI protein with known methods according to the base sequence described in the present invention since there are various textbooks available on the culturing of a transformant. Here, any one of bacterium such as E. coli, yeast, and animal cell is usable as a host, but the animal cell is especially preferable. For introducing a gene into the cell, the ribosome method, the electroporation method and the like can be used. In particular, a manupilation for microinjection into a nucleus is preferably used.

For purifying the human HRPI protein or the mutant of the human HRPI protein from the resultant cultured products, a suitable method may be selected from various purifying methods, such as immunoprecipitation, salting out, ultrafiltration, isoelectric precipitation, gel filtration, electrophoresis, ion-exchange chromatography, various affinity chromatographies such as hydrophobic chromatography and antibody chromatography, chromatofocusing, adsorption chromatography, and a reverse phase chromatography.

The human HRPI protein or the mutant of the human HRPI protein to be manufactured may be prepared as a fused peptide with the other polypeptides by a transformant in the manufacturing step. In this case, it is necessary to cleave the human HRPI protein or the mutant of the human HRPI protein in the purifying process by processing with a chemical material such as bromocyanogen or an enzyme such as protease.

The present invention discloses, about the antigenicity of human HRPI protein, that an antibody is easily obtained by immunizing the human HRPI protein obtained by the abovementioned method, or by immunizing oligopeptide consisting of an amino acid sequence specific for the human HRPI protein, as shown in Example 3, with animals except humans. Therefore, the antibody (hereinafter sometimes referred to as human HRPI antibody) that recognizes the human HRPI protein of the present invention is obtained by immunizing the human HRPI protein with an animal except humans and the animals from which the human HRPI protein is derived. Such antibody includes in its scope the antibodies for which it can be proved, by Western blotting method, ELISA method or an immunostaining method (e.g., the tissue staining of a frozen specimen and a paraffinized specimen), that the antibodies recognize the human HRPI proteins.

Employing, as an immunogen, part of the protein or part of the protein bound to a carrier protein such as bovine serum albumin is a method in common use by those skilled in the art. The part of the protein may be synthesized using a peptide synthesizer, for example. The part of the protein should be preferably 8 amino acid residues or more.

As for substances whose antigenicity has been elucidated, it is generally known that, if polyclonal antibodies against them can be obtained by immunization, monoclonal antibodies can be produced by hybridomas using lymphocytes of the immunized animal (e.g., see "Antibodies, A Laboratory Manual" (Cold Spring Harbor Laboratory Press, 1988) Chapter 6). Thus, the human HRPI antibody of the present invention includes a monoclonal antibody in the scope.

In the present invention, the antibodies also embrace active fragments thereof. The term "active fragment" means the fragment of the antibody that possesses the antigen-antibody reactivity; and more specifically named are F(ab')₂, Fab', Fab, Fv, etc. For example, F(ab')₂ results when the antibody of the present invention is digested with pepsin; and Fab results when digested with papain. Fab is obtained when F(ab')₂ is reduced with a reagent such as 2-mercaptoethanol and alkylated with monoiodoacetic acid. Fv is an active fragment of a monovalent antibody wherein a heavy chain variable region and a light chain variable region are bonded to each other via a linker.

Chimeric antibodies may be obtained by retaining these active fragments, while substituting fragments from other animals for the other parts.

For detecting human HRPI, there are methods using antibodies and methods using enzyme reaction.

As for the methods using antibodies, there are (1) a method of detecting human HRPI using the labeled human HRPI antibodies, and (2) a method of detecting human HRPI using human HRPI antibodies and labeled secondary antibodies to the antibodies. The labels are, for example, radioisotopes (RI), enzymes, avidin or biotin, and fluorescence materials (FITC or rhodamine).

As methods of using enzyme reaction, there are, for example, ELISA method, immunoagglutination method, and a method of identifying immunoreacted molecules by means of Western blotting, and other similar methods.

The present invention is explained in detail by the examples shown below.

### [Example 1]

Determination of human HRPI base sequence and human HRPI amino acid sequence.
1. The base sequence that encodes the amino acid sequence homologous to the sequence of rat HRPI gene presented in SEQ ID NO: 3 in the Sequence Listing was searched from the dbEST by using TBLASTN (http;//www.genome.ad.jp). As a result, EST registered under the numbers of W88946, H94887, AA001877 and W89062 were extracted. From the sequences obtained by connecting these EST, the following four kinds of primers were prepared using a DNA synthesizer (Type 392, ABI Corporation):
Primer 1: CTG GCA TTT TGG TGG TAA TGA GAG (the base sequence set forth in SEQ ID NO: 4 of the Sequence Listing)
Primer 2: TCA GAC CTT TCC CAC CTG CTG TCT C (the base sequence set forth in SEQ ID NO: 5 of the Sequence Listing)
Primer 3: GGG AAT AGA TGA CAT ACG TCA CAA G (the base sequence set forth in SEQ ID NO: 6 of the Sequence Listing)
Primer 4: GCT GTA GTC TAC ATG AGA ATA GAC (the base sequence set forth in SEQ ID NO: 7 of the Sequence Listing)

2. Using the Marathon cDNA library of human fetal liver (Clonetech Corporation) and according to its manual, PCR was carried out with the primer 1 and an adapter sequence, and then, with respect to the resultant PCR product, PCR was carried out with the primer 2 and an adapter sequence. Also, using the same cDNA library, PCR was carried out with the primer 3 and an adapter sequence; thereafter with respect to the resultant PCR product, the PCR was carried out with, the primer 4 and an adapter sequence.
3. The PCR products obtained as described above were separated by electrophoresis on 0.7% agarose gel, and a target amplified DNA band was excised therefrom. The amplified DNA was purified with Gene Clean (Bio 101 Corporation). Then, using a TA Cloning Kit (Invitrogen Corporation), the purified DNA was incorporated into the pCRII vector (accompanying the kit).
4. The vector prepared in step 3 above was mixed with E. coli (JM109). After allowing it to react for 30 minutes on ice, for 45 minutes at 42°C, and then for three minutes on ice, 900 µl of the SOC culture medium was added thereto, and it was left for one hour at 37°C; thus, the vector was introduced to the E. coli (JM109). The E. coli was cultured in an LB agar culture medium (containing 100 µg/ml Ampicillin, 0.1 mM IPTG, and 0.004%X-gal) for 16 hours at 37°C. Among the colonies thus formed, white colonies were planted to 2 ml of an LB culture medium (containing 100 µg/ml Ampicillin), and cultured for 16 hours at 37°C.
5. Thereafter, the cultured transformants were collected by a centrifugal separation at 12000 rpm for one minute. The plasmid DNA solution was recovered according to Magic Miniprep (Registered trademark, Promega Corporation).
6. The base sequence of the recovered DNA was determined using the Dye Terminator FS Sequencing Kit (Perkin-Elmer Corporation) in the following steps:
(1) A sequence sample was prepared from the recovered DNA according to the manual of the kit.
(2) PCR of the DNA was carried out according to the manual of the kit in the following composition system:

| | |
|---|---|
| Template DNA | 600 µl |
| Primer DNA | 3.2 pmol |
| Sequence mix | 8 µl |

to which sterilized and distilled water was added to make a reactant liquid of 20 µl in total.
(3) Extraction of the PCR reactant liquid was carried out with phenol/chloroform.
(4) The extracted product was precipitated by addition of ethanol for purification and dried.
(5) The resultant product was dissolved in a solution of 3 µl containing 3% blue dextran, 50 mM EDTA, and 80% formamide, and denatured for 2 minutes at 95°C, and then cooled with ice; thus a sample for the sequence was obtained.
(6) The sample thus obtained was analyzed with 373 Stretch DNA Sequencer (Parkin-Elmer Corporation) and the base sequence thereof was determined. The base sequence is shown in SEQ ID NO: 2 of the Sequence Listing.
7. From the base sequence set forth in SEQ ID NO: 2 the primers having the following sequences were prepared using a DNA synthesizer:
- Primer 5:: CAG AAC TCA TCA ACT GGA AAC AGC (the base sequence set forth in SEQ ID NO: 8 of the Sequence Listing)
- Primer 6:: CTC TGG CTC CTC TCA TTA CCA CCA (the base sequence set forth in SEQ ID NO: 9 of the Sequence Listing)

8. With respect to human liver cDNA library, PCR was carried out using the primers 5 and 6.
9. The resultant PCR products (cDNA) were treated with the similar operations as described in steps 3 to 7 above, and the base sequence thereof was determined. The base sequence was matched with that of SEQ ID NO. 2 of the Sequence Listing. Consequently, the cDNA was recognized as a full length human HRPI cDNA.
10. An amino acid sequence of human HRPI was determined from the base sequence of SEQ ID NO: 2 of the Sequence Listing. The amino acid sequence is shown in SEQ ID NO: 1 of the Sequence Listing.
11. The human HRPI cDNA was inserted into pCR II vector, and the vector was introduced into E. coli, JM109, whereby a transformant was prepared. The transformant was deposited with the National Institute of Bioscience and Human-Technology, Agency of Industrial Science and Technology (located at 1-3 Higashi 1-chome, Tsukuba-shi, Ibaraki-ken, Japan) on March 5, 1998. This deposit was made according to the Budapest Treaty. The deposit number is FERM BP-6285.

The base sequence that is homologous to the base sequence set forth in SEQ ID NO: 2 of the Sequence Listing was searched from the dbEST by using BLASTN (http;//www.genome.ad.jp/SIT/BLAST.html). As a result, EST registered under the number of AF023466 was extracted. The amino acid sequence of human HPRI contains AF023466 and has a length 1.8 times longer than that. The base sequence of human HPRI gene is homologous to AF 023466 and its open reading frame is 1.8 times as long as that. Naturally, a longer base sequence or amino acid sequence cannot be assumed from the base sequence or amino acid sequence of AF023466.

### [Example 2]

### Expression of human HRPI protein

### 1. Construction of recombinant vector

FLAG-histidine tag was incorporated into human HPRI cDNA, which was integrated into pET3a. The details thereof are as follows:
(1) The pCR II vector having human HRPI cDNA inserted therein as described in step 11 of Example 1 was amplified by the PCR method described in "Current protocols in molecular biology" (Green Publishing Associates and Wiley-Interscience, 1987), Chapter 15. Primers 7 and 8 having the following base sequences were used in the amplification, and the base sequence which encodes FLAG-histidine tag to be incorporated into the human HPRI gene and the first methionine portion of the human HRPI gene was amplified as the NdeI recognition site:
Primer 7: CAT ATG GAC TAC AAG GAC GAC GAT GAC AAG CAT CAC CAT CAC CAT CAC ATG TTA CCA TTG CAA GGT GCC CAG (the base sequence set forth in SEQ ID NO: 10 of the Sequence Listing)
Primer 8: GGA TCC TTA GAG AGG TAC CCA GTT CCA CTG G (the base sequence set forth in SEQ ID NO: 11 of the Sequence Listing)

(2) The amplified gene was electrophoresed on 0.7% agarose gel. Thereafter a band near about 1kb was cut out therefrom, and then it was purified with Quiaquick gel extraction kit (Quiagen, Inc.). Then, using TA Cloning Kit (Invitrogen Corporation), the purified DNA was incorporated into pCRII vector.
(3) DNA was prepared from the vector by the method described in steps 4 to 6 of Example 1 and the base sequence thereof was confirmed.
(4) From the DNA thus obtained, human HPRI DNA in which FLAG-histidine tag is incorporated was cut out with NdeI and BamHI by allowing it to react for three hours at 37°C in a solution having the following compositions:

| | |
|---|---|
| HRPI DNA (1 µg/µl) | 5 µl |
| NdeI (Takara Shuzo, Inc.) | 1 µl |
| BamHI (Takara Shuzo, Inc.) | 1 µl |
| 10-fold K buffer (Takara Shuzo, Inc.) | 5 µl |
| Sterilized and distilled water | 38 µl |
| Total | 50 µl |

(5) The DNA thus cut out was electrophoresed on 0.7% agarose gel. Thereafter a band near about 1kb was cut out therefrom, and then it was purified with Quiaquick gel extraction kit (Quiagen, Inc.). The purified DNA solution thus obtained was designated as "solution A".
(6) pET3a vector was digested with NdeI and BamHI in the same manner as described in (4) above. The resultant vector solution was designated as "solution B".
(7) To the solution B, 5 µl of 1 M Tris-HCl pH8.0 and 1 µl of BAP (Takara Shuzo, Inc.) were added, and the dephosphorylation thereof was performed for 30 minutes at 65°C and subsequently for one hour at 37°C.
(8) The vector thus dephosphorylated was subjected to phenol-chloroform treatment three times and the supernatant thereof was collected.
(9) Thereto added were 5 µl of 3M NaOAc (pH5.2) and 130 µl of -20°C ethanol and thereby the vector was subjected to ethanol precipitation, and then centrifuged for 20 minutes at 15000 r/min.
(10) After the precipitate (the vector) was washed with 70 % ethanol, it was dehydrated. Then, the dehydrated vector was dissolved into 40 µl of sterilized water. The solution thus obtained was designated as "solution C".
(11) Solution A and solution C were mixed to give the following composition, which was allowed to react for one hour at 16°C using Ligation kit Ver.2 (Takara Shuzo, Inc. ), and the human HRPI DNA to which FLAG-histidine tag had been added was introduced into the pET3a.

| | |
|---|---|
| Solution A | 3 µl |
| Solution C | 1 µl |
| Sterilized water | 6 µl |
| Ligation Kit Ver.2 | 10 µl |
| Total | 20 µl |

(12) DNA was prepared from the pET3a vector by the method described in items 4 to 6 of Example 1 except that neither IPTG nor X-gal was added to the LB agar culture medium, and the base sequence thereof was confirmed.

### 2. Expression of the human HRPI to which FLAG - histidine tag is added

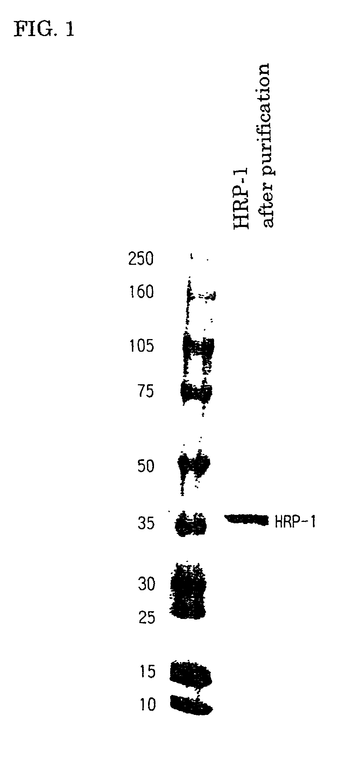

(1) The FLAG- histidine tag HRPI cDNA that was incorporated into the pET3a as described above was introduced into E. coli BL21 (DE3) by the following operation.
(2) The E. coli prepared in (1) above was cultured in LB culture medium containing 100 µg/ml of Ampicillin. When its turbidity measured with a spectrophotometer (Beckman) reached 0.5 at a wavelength of 600 nm, IPTG was added to give a concentration of 0.5mM, thereby inducing the expression of HRPI protein.
(3) Two hours later, E. coli cells were recovered, and after suspension in Lysis Buffer, they were sonicated. After that, they were centrifuged at 18000 r/min, at 4°C for 15 minutes with 50.2Ti rotor using Beckman Optima XL-80.
(4) The precipitate was dissolved in 6 M guanidine hydrochloride and purified with Ni-agarose (Quiagen) in accordance with its instruction manual.
(5) The purified protein was subjected to SDS-polyacrylamide electrophoresis and stained with Coomassie brilliant blue. The result is shown in Fig. 1. A band was seen in the position of about 35 kD, which demonstrated that purification was successful.

### [Example 3]

### Production of antibody recognizing human HRPI protein

### 1. Production of antigen

(1) The following peptide A and peptide B were synthesized.
   Peptide A: peptide consisting of 26 amino acids from the 73rd (i.e.,Thr) to the 98th (i.e.,Trp) of the amino acid sequence of the human HRPI protein set forth in SEQ ID NO: 1 of the Sequence Listing;
   Peptide B: peptide consisting amino acids, in which cysteine was added to the N-terminus of 25 amino acids from the 185th (i.e., Ile) to the 209th (i.e., Thr) of the amino acid sequence of the human HRPI protein set forth in SEQ ID NO: 1 of Sequence Listing.

   The cysteine was added for the conjugation with Hemocyanin from Macroschisma Sinensis (KHL). The synthesizing work was entrusted to Iwaki Glass Corporation.
   The synthesized peptides of 2 mg were conjugated with 2 mg of maleimide-activated KLH (Pierce Inc). The reaction was carried out according to the method described in the instruction manual attached to the kit of Pierce Inc.
(2) The human HRPI protein produced in Example 2 was prepared in 1 ml at a concentration of 1mg/ml, which was used as antigen.

### 2. Immunization

100 µl of antigen (peptide A, peptide B or the human HRPI protein) solution (1mg/ml), 0.5 ml of PBS, and 0.5 ml of Freund complete adjuvant (Difco, Inc.) were taken in a syringe and mixed to emulsion, which was subcutaneously inoculated on four points of the back of a rabbit.

One week later, the second immunization was carried out. From the second time, the adjuvant used was changed to the Freund incomplete adjuvant (Difco Inc.). The other manipulations were the same as the first time.

From the second time, immunizations were done at one week intervals, six times in total.

### 3. Preparation of antibodies

One week after the last immunization, blood was collected from the rabbit. The blood was allowed to stand at room temperature for three hours. After sufficiently coagulated, the blood was centrifuged for five minutes at 3,000 r/min, and the supernatant (serum) was recovered.

The serum was salted out by adding saturated ammonium sulfate to bring the last concentration to 50 % concentration.

This sample was centrifuged and fractions containing antibodies were precipitated. Subsequently, the precipitate was dissolved in PBS and further salted out against PBS.

Then, with respect to the salted out product of the serum obtained from the rabbit to which a peptide antigen was administrated, the antibodies were purified by affinity chromatography using a Protein G Sepharose column (Registered Trademark, Amarsham Pharmacia Biotech Inc.). As a result, a total amount of 370 mg of an IgG fraction was obtained.

The aforementioned IgG fraction and the salted out product of the serum obtained from the rabbit to which human HRPI protein was administrated were respectively purified by affinity chromatography using the column prepared by binding the peptides or proteins, which were used in the immunization, to NHS-activated Sepharose (Amersham Pharmacia Biotech Inc.) according to the instruction manual attached to the Sepharose. As a result, 5 mg of purified antibodies were obtained in total.

### 4. Determination of antibody titers

Titers of the purified antibodies thus obtained were determined by ELISA.
(1) Each of the antigen solutions (solution of peptide A, peptide B or human HRPI protein) was dissolved in PBS to give a concentration of 25 µg / ml, which was dispensed into each well of a 96-well ELISA plate (Xenobind, registered trademark, Xenopore Inc.) and allowed to stand overnight at 4°C.
(2) The antigen solution was discarded and each 200 µl of Blockace (Registered Trademark, Dainippon Pharmaceuticals Co., Ltd.) diluted fourfold with distilled water was dispensed into each well. Blocking was carried out by allowing it to stand at room temperature for two hours.
(3) The blocking solution was discarded and the purified antibodies were indivisually added as a primary antibody. The solutions of the purified antibodies, which were diluted with PBS serially in twofold such that the respective concentration of the solutions could successively reach 10 µg/ml, 5 µg/ml, 2.5 µg/ml, etc. from the first row to the twelfth of the 96 wells, were respectively poured by 50 µl into each well. The concentration of the antibodies in the twelfth well was about 0.5 µg/ml. Here, IgG purified from the blood of a rabbit that was not immunized was used as a control. The antibody solutions thus poured into the wells were allowed to react at room temperature for one hour.
(4) The antibody solutions were discarded and the plate was washed with 0.05% Tweed 20/PBS four times. Then, each 50 µl of the biotinylated anti-rabbit IgG antibody (Vector Inc.) that had been 1000-fold diluted with PBS was dispensed poured into each well as a secondary antibody solution. It was allowed to react at room temperature for 30 minutes.
(5) After the secondary antibodies were discarded, the plate was washed with 0.05% Tweed 20/PBS four times, and each 50 µl of ABC solution diluted 1000-fold (Vector Inc.) was poured into each well. It was allowed to stand at room temperature for 30 minutes.
(6) After the ABC solution was discarded, the plate was washed with 0.05% Tweed 20/PBS four times, and each 100 µl of OPD (orthophenylenediamine)-H₂O₂/PBS was dispensed to each well. After the reaction was quenched with 2N sulfuric acid upon sufficient coloring, the absorbance at 490 nm was measured with a microplate reader (Bio-rad Inc.).

As a result, with respect to both of the antibodies obtained by administrating peptide A and those obtained by administrating peptide B their titers were greater in comparison with the control.

### 5. Western Blotting

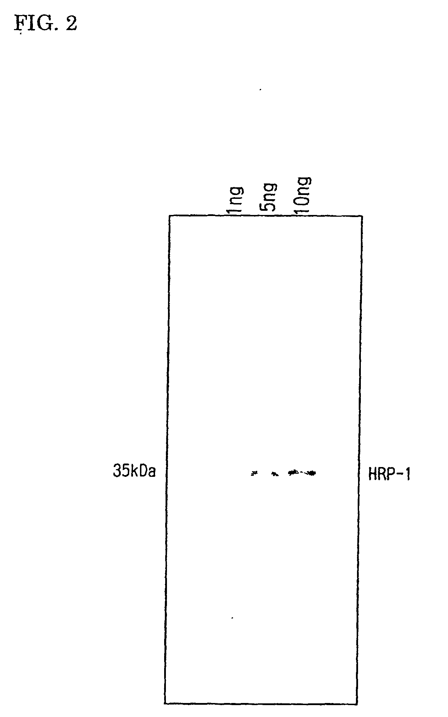

Western blotting was carried out using Proto Blot II AP system (Promega Inc.) and the antiserum which was obtained by administrating a human HRPI protein and which was 10000-fold diluted. The results are shown in Fig. 2. As shown in Fig. 2, it was possible to detect human HRPI protein of 1 ng even when the antiserum was diluted 10000-fold (1 ng / 10 µl).

### [Example 4]

### Northern blotting

### 1. Preparation of probe

The coding region part of human HRPI cDNA that exhibits its base sequence at SEQ ID NO. 2 of Sequence Listing was amplified with PCR and labeled to make a probe of Northern blotting. The manipulation is shown below.

### (1) Synthesis of primer

The primers 7 and 8 used in Example 2 were individually prepared to 20pmol/µl with distillation water. These were used as a PCR primer for performing the following PCR manipulations.

### (2) PCR

PCR was carried out, employing vector pET3a into which Human HRPI cDNA was introduced as the template of PCR, and using primer 7 and primer 8, and Takara Taq pol (Registered trademark, Takara Shuzo Inc.) under the following conditions.

They were allowed to stand at 94°C for one minute.

Subsequently, a cycle of "at 94°C for 45 seconds, followed by at 60°C for two minutes, and followed by at 72°C for two minutes" was repeated 30 times. Then, after allowing them to stand at 4°C, the PCR manipulations were completed.

The composition of the PCR reaction solution was as follows:

| | |
|---|---|
| 0.5 ng/µl human HRPI pET3a | 1 µl |
| Taq pol (Takara Shuzo, Inc.) | 0.1 µl |
| 10-fold concentration PCR buffer (Takara Shuzo, Inc.) | 2 µl |
| dNTP mix (Takara Shuzo, Inc.) | 1.6 µl |
| Primer 7 | 2 µl |
| Primer 8 | 2 µl |
| sterilized distilled water | 11.3 µl |
| Total | 20 µl |

PCR product was electrophoresed on 0.7 % agarose gel.

From the gel PCR product (hereinafter, referred to as "fragment A") was cut, and the fragment A was recovered with Gene Clean (Bio 101 Corporation), and its band was checked with mini-gel electrophoresis. As a result, the concentration of the DNA solution of fragment A was estimated to be 25 ng/µl.

### (3) Labeling

Sterilized distilled water of 4 µl was added to 5 µl of DNA solution of fragment A. It was allowed to stand at 98 °C for 10 minutes. Then, fragment A was denatured by cooling it on ice.

After placing the denatured fragment A in a solution of the following composition at 37°C for 30 minutes, and then at 65°C for 10 minutes, it was labeled using a random labeling kit (made by Boehringer Mannheim).

| | |
|---|---|
| denatured fragment A | 9 µl |
| dA, G, TTP mix (Boehringer Mannheim) | 3 µl |
| reaction solution mix (Boehringer Mannheim) | 2 µl |
| d-32P-dCTP (Boehringer Mannheim) | 5 µl |
| Klenow fragment solution (Boehringer Mannheim) | 1 µl |
| Total | 20 µl |

Labeled fragment A was purified with this solution by G-50 micro spin column. Packing was performed by turning the column for one minute at 3000 r/min with a swing rotor. A labeled fragment A solution was added to the column by 20 µl, and the column was turned for two minutes at 3000 r/min, and label fragment A was separated by eluting it with T50E.

### 2. Hybridization

### (1) Prehybridization

Membranes (human MTN (Registered trademark, Clone Tech. Inc.)) to which mRNA was fixed were put in 10 ml of hybridization solution having the following compositions, and it was allowed to stand at 42 °C for 150 minutes, and blocking was carried out on the membranes with denatured salmon sperm DNA.

### (Hybridization solution)

5-fold concentration SSPE
10-fold Denhartd's solution
2% SDS, 50% formamide
100 µg/ml denatured salmon sperm

### (2) Hybridization

Labeled fragment A solution of 7 µl was added to 20 ml of the hybridization solution and it was stirred well to mix.

The hybridization solution of 5 ml each was added to each square laboratory dish, and one sheet each of the above membranes was put in each laboratory dish. A vinyl sheet was placed each membrane, and hybridization was carried out at 42°C overnight (about 15 hours).

The hybridization solution was discarded, and a washing solution (2-fold concentration SSC, 0.1 % SDS) was added to the laboratory dish and washing was carried out for 5 minutes at room temperature. Such washing was repeated four times.

### (3) Autoradiogram

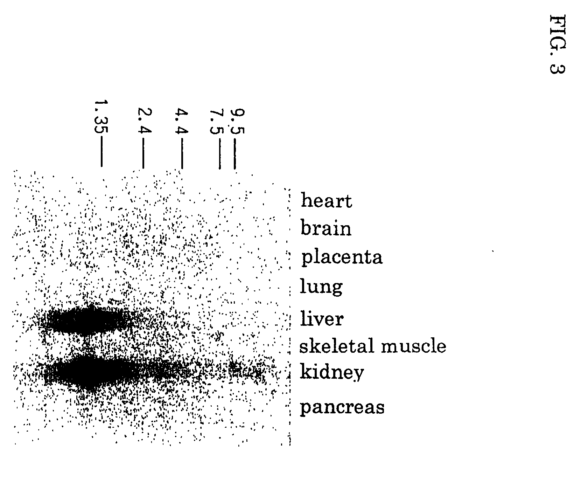

These membranes were exposed to radiosensitive paper for 16 hours to obtain an autoradiographic image. The results are shown in Fig. 3. As can be seen in Fig. 3, human HRPI mRNA was expressed in the liver and kidney, but not in the other organs (heart, brain, placenta, lung, skeletal muscle, and pancreas). Expression was examined with respect to other organs, but the expression of human HRPI mRNA could not be found in the organs except the liver and the kidney.

### Possibility for industrial application

According to the present invention, human HRPI, the mutants thereof, the partial proteins thereof and the polynucleotides (including the polynucleotides which consist of the base sequences set forth in SEQ ID NO: 2 of the Sequence Listing) which encode human HRPI, the polynucleotides which are a part thereof, and the polynucleotides that are the antisense strand thereof are useful for explicating the mechanism of carsinogenesis.

Furthermore, though in the present patent application specifications it is not explained in detail, based on an experiment using rat HRPI antibodies, the present inventors found that human HRPI is localized in centrosome. Therefore, human HRPI is useful for the research of the cell cycle as well as the research of the factor controlling cell division.

The antibody of the present invention can be used for explicating a mechanism of carsinogenesis and the diagnosis thereof. Furthermore, it will be usable in the research of cell cycle as well as the research of the factors controlling the division of cultured liver cells.

## Claims

1. A protein having at least the amino acid sequence set forth in SEQ ID NO: 1 of the Sequence Listing.

2. A protein consisting of an amino acid sequence in which one or more amino acids set forth in SEQ ID No: 1 of the Sequence Listing are substituted or deleted, or in which one or more amino acids are added to the amino acid sequence of SEQ ID NO: 1 of the Sequence Listing.

3. A polynucleotide that encodes the protein according to Claim 1 or 2.

4. A polynucleotide consisting of the base sequence set forth in SEQ ID NO: 2 of the Sequence Listing.

5. A polynucleotide that is part of the polynucleotide set forth in Claim 3 or 4 and which comprises at least 12 or more continuous bases.

6. A polynucleotide which is part of the antisense polynucleotide consisting of the base sequence of the antisense strand of the polynucleotide set forth in Claim 3 or 4 and which comprises at least 12 or more continuous bases.

7. A derivative of the polynucleotide set forth in Claim 5 or 6.

8. A polynucleotide set forth in any one of Claims 5 to 7, said polynucleotide being chemically modified.

9. An antibody that recognizes the protein set forth in Claim 1 or 2.
